# EUROPEAN PATENT SPECIFICATION

(11) **EP 2 262 552 B1**
(45) Date of publication and mention of the grant of the patent: **25.12.2013**
(21) Application number: 09720643.7
(22) Date of filing: 06.03.2009
(51) Int. Cl.: A61M 5/142, H02J 7/00, H02J 7/34, H02J 9/06

(54) **MEDICAL DEVICE WITH AT LEAST TWO ENERGY SOURCES**
MEDIZINISCHE VORRICHTUNG MIT MINDESTENS ZWEI ENERGIEQUELLEN
DISPOSITIF MÉDICAL AVEC AU MOINS DEUX SOURCES D'ÉNERGIE

(30) Priority: 10.03.2008 EP 08004343
(43) Date of publication of application: 22.12.2010
(73) Proprietor: Roche Diagnostics GmbH, 68305 Mannheim (DE); F. Hoffmann-La Roche AG, 4070 Basel (CH)
(72) Inventor: DOGWILER, Joerg, CH-8962 Bergdietikon (CH); KAUFMANN, Heiner, CH-3008 Bern (CH); KIPFER, Urs, CH-3432 Lützelflüh-Goldbach (CH); SIGRIST, Reto, 3207 Golaten (CH)
(74) Representative: Rentsch Partner AG
(86) International application number: PCT/EP2009/001596
(87) International publication number: WO 2009/112201

(56) References cited:
- EP-A- 0 982 830
- WO-A-00/52807
- WO-A-97/13310
- WO-A-2007/129317
- DE-A1- 3 834 001
- US-A- 3 789 854
- US-A- 6 137 261
- US-A1- 2003 160 588
- US-A1- 2005 248 313

## Description

The invention relates to a medical device according to claim 1 and to a medical system according to claim 17. In particular, the invention relates to a medical device for diabetes treatment and to a medical system comprising such a device. The medical device may, for example, be a medical dosing device, such as an insulin pump, or a blood glucose meter.

Typically, portable medical devices are powered by non-rechargeable batteries.

The document WO 2000/52807 A1 discloses a battery pack comprising a plurality of battery cells, each of them constituting an energy source. Depleted battery cells are switched with charged battery cells via a battery switching circuit if the monitored voltage falls below a predetermined level.

The document EP 0982830 A2 discloses a battery pack with a plurality of cells which are, together with a protective circuit, encased in a container. The protective circuit comprises a cell failure detector for each of the cells and a cell interrupter to disconnect a faulty cell.

It is an object of the invention to provide a robust and reliable medical device comprising a rechargeable energy supply that is fail safe or single fault safe.

In order to implement this and still further objects of the invention, which will become more readily apparent as the description proceeds, a medical device is provided that has an energy supply and a control unit for monitoring and controlling the energy supply. The energy supply comprises at least two energy sources, of which at least one energy source constitutes a backup energy source. The at least one energy source that constitutes the backup energy source may also be used for supplying energy in cases where no backup is needed for the one or more other energy sources. The control unit is designed such that it can detect a fault in the energy sources and can disconnect the faulty energy source upon fault detection.

Hence, the medical device according to the invention constitutes a redundant system that is fail-safe or single fault safe. If one of the energy sources fails, then the other or one of the other energy sources will function as backup energy source and either take over the function of the faulty energy source or provide enough energy to serve as emergency energy supply for triggering an alarm. Thus fail-safe operation of the medical device is ensured even if a fault occurs in one of the energy sources of the energy supply.

The control unit preferably comprises means for measuring and evaluating the output voltage and/or current of each energy source and means for disconnecting each energy source from the rest of the medical device, i.e. the load of the energy supply. In particular, the control unit comprises a control subunit for each energy source, wherein each control subunit has means for measuring and evaluating the output voltage and/or current of its associated energy source and means for disconnecting the associated energy source from the load. In case of the medical device being an insulin pump, the load is basically the actual pump.

The energy supply of a medical device according to the present invention is rechargeable, wherein the energy supply comprises a lithium(-ion) polymer battery with at least two battery stacks. Each battery stack constitutes one energy source and has a terminal or contact on positive potential and a terminal or contact on negative potential. Each battery stack may furthermore comprise one or more battery cells, in particular one or more battery bi-cells. A bi-cell is defined as a cell with one anode and two cathodes, the cathodes being positioned on either sides of the anode. The height of a bi-cell lies, for example, in the range of 600 to 800 micrometers.

Lithium(-ion) polymer batteries are robust to physical damage and can be cost-efficiently manufactured. Furthermore, as lithium(-ion) polymer batteries are rechargeable the user does not have to purchase a new energy supply once the original one has been discharged. The energy provided by a lithium(-ion) polymer battery per volume is basically optimal, which broadens application/device integration possibilities and possibly prolongs the running time. Due to the polymeric nature no rigid casing is required, so that lithium(-ion) polymer batteries can be specifically shaped to fit the medical device they shall supply with energy. This has the advantage that the space that is provided by a medical device for an energy supply can be optimally utilized. For a given shape optimal capacity of the energy source can be provided. Furthermore, as lithium(-ion) polymer batteries can be manufactured to be very thin, also the medical devices they power such as insulin pumps can be made very thin, so that they can be more discreetly employed/used by a user. Further, as lithium(-ion) polymer batteries have basically optimal gravimetric density, the weight of the medical device can be optimized, i.e. reduced, leading to an easier-to-carry medical device. Also due to basically optimal volumetric density of the lithium(-ion) polymer batteries longest possible running time of the energy supply of the medical device can be ensured. The energy supply with lithium(-ion) polymer batteries is generally robust, i.e. tolerant, with respect to temperature and power variations in particular due to small internal resistance. As the cell-voltage and the discharge voltage range (typically 4.2 Volt - 3.0 Volt) of a lithium(-ion) polymer battery are rather high, the energy supply and the energy source, respectively, are little susceptible to contamination of their terminals/contacts.

In the context of a medical device, and in particular a portable device continuously carried by a user and being required to be fail-safe or single fault safe, such as an insulin pump, an energy supply according to the present invention shows particular advantages. Providing a lithium (-ion) polymer battery with at least two battery stacks according to the present invention results in an energy supply which is compact and easily exchangeable and/or rechargeable as one compact unit while at the same time meeting the fault safety requirements for this kind of devices.

It's a further object of the invention to provide a medical system, the medical system comprising a medical device as described above and a charging station for an energy supply of a medical device as described above.

The charging station according to the invention comprises as energy supplying means an USB-plug, a line connector (also called network connector), a solar cell panel, a primary battery, a plug for a car cigarette lighter outlet, a connector for a mobile phone accumulator and/or a dynamo with a crank handle. Preferably at least two different energy supplying means are provided. By providing several different types of energy supplying means, the user is not restricted to one type of energy (e.g. a primary battery) but can switch to a different kind of energy, in particular to a different kind of primary energy, (e.g. solar energy or the energy provided by a car battery via a cigarette lighter outlet) which is available at that moment. This enhances the independency of the user from purchasable and often quite expensive primary batteries and guaranties substantially permanent and uninterruptible energy availability also in non-everyday situations like on vacation, on travel or in free time. Hence, the charging station according to the invention gives the user more individuality and flexibility. The user can charge the energy supply of his medical device according to his needs, which makes the charging individual, and according to the energy resources at hand (i.e. according to availability). The user can quickly adapt to outer circumstances, e.g. if he is in the office he may e.g. use the line connector or the USB-plug, on vacation he may e.g. use solar energy or the dynamo by rotating the crank handle etc.

It should be noted that the charging station as described above may be considered as distinguishable invention which the applicant reserves the right to pursue separately from the present applications.

Further advantageous features and applications of the invention can be found in the dependent claims as well as in the following description of the drawings illustrating the invention. In the drawings like reference signs designate the same or similar parts throughout the several figures of which:
Fig. 1 shows a schematic drawing of an embodiment of a medical device according to the invention,
Fig. 2 shows a schematic drawing of a further embodiment of a medical device according to the invention,
Fig. 3 shows a schematic drawing of an embodiment of an energy supply,
Fig. 4 shows a schematic drawing of a further embodiment of an energy supply supporting a reservoir for a fluid,
Fig. 5 shows a schematic drawing of a further embodiment of an energy supply with two energy sources,
Fig. 6 shows a schematic drawing of a further embodiment of an energy supply with two energy sources,
Fig. 7 shows a schematic drawing of a further embodiment of an energy supply with two energy sources,
Fig. 8 shows a schematic drawing of a further embodiment of an energy supply with two energy sources,
Fig. 9 shows a block diagram of a medical device according to the invention, and
Fig. 10 shows a schematic drawing of a charging station of a medical system according to the invention.

The depicted medical devices may for example be medical dosing devices such as insulin pumps.

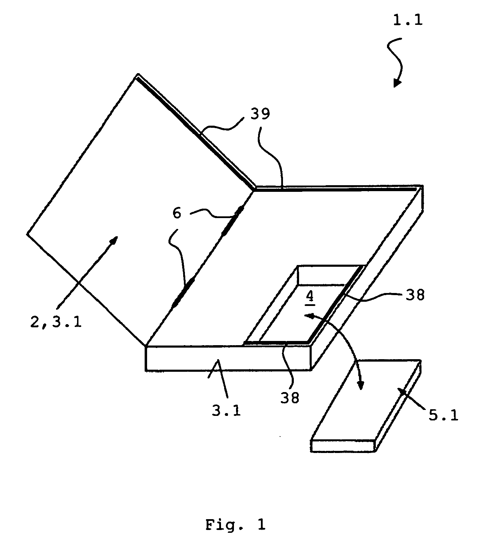

Figure 1 schematically shows a first embodiment of medical device 1.1 with a top cover 2. The top cover 2 forms part of the housing 3.1 of the medical device 1.1. The top cover 2 is preferably mounted pivotally on hinges 6. The medical device 1.1 has a compartment 4 for a replaceable energy supply 5.1 being designed as will be described below in more detail. The replaceability of the energy supply 5.1 is indicated in Figure 1 by the bent arrow. In the embodiment depicted in Figure 1 the energy supply 5.1 is placed in the compartment 4 and, hence, into the housing 3.1 of the medical device 1.1. Therefore, no specific sealing functions have to be performed by the outer surfaces of the energy supply 5.1/compartment 4 and no specific sealing or gaskets have to be provided at the contact surfaces of the energy supply 5.1/compartment 4. The sealing function is performed by the top cover 2 or housing 3.1, see exemplarily the sealing or gaskets 39 depicted in Figure 1. Of course, there may be specific sealing or gaskets 38 arranged at contact surfaces of the compartment 4 for further sealing.

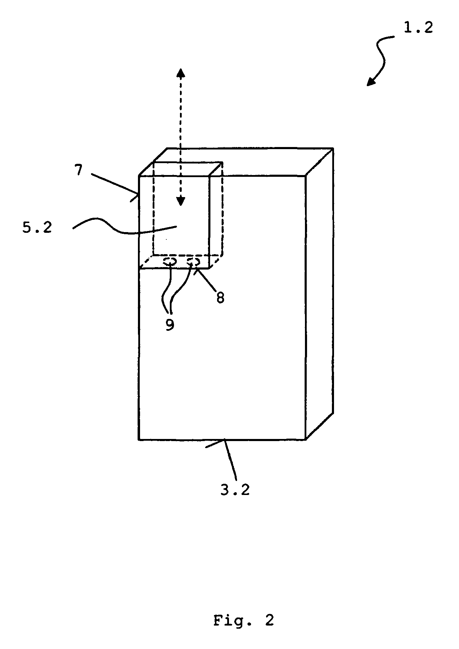

Figure 2 shows another embodiment of a medical device 1.2 with a replaceable energy supply 5.2 being designed as will be described below in more detail. The dashed arrow indicates how the energy supply 5.2 can be exchanged. In the embodiment of Figure 2 part of the housing 3.2 of the medical device 1.2 is formed by a housing 7 of the energy supply 5.2. For sealing purposes the contact surface 8, which is the outer surface with the terminals 9 (also called contacts 9) of the energy supply 5.2, is preferably provided with a sealing/one or more gasket, in particular to protect the medical device 1.2 against humidity. That is, the terminals 9 are sealed. For simplicity, only two terminals 9 are shown. Due to the sealing/gasket it is ensured that the medical device 1.2 conforms to IPX8 (international protection rating X8), i.e. the medical device 1.2 is protected against harmful ingress of water up to and beyond 1 meter. In detail the medical device 1.2 is suitable for continuous immersion in water under conditions which shall be specified by the manufacturer (confer http://en.wikipedia.org/wiki/IP_Code).

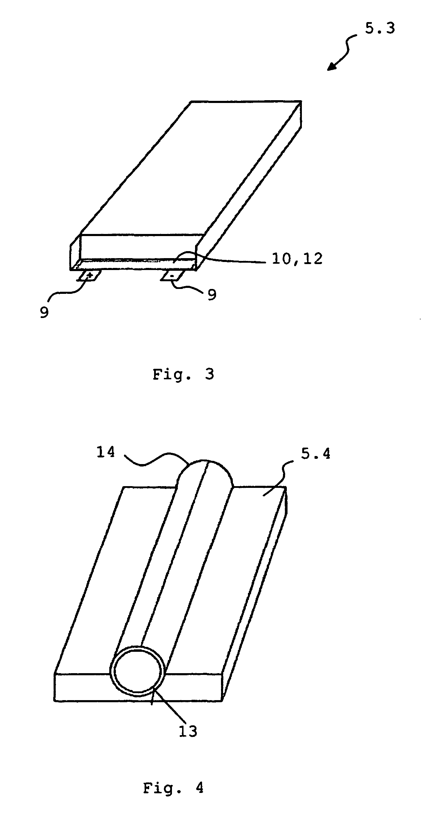

Figure 3 depicts an energy supply 5.3 (corresponding to the energy supplies 5.1 and 5.2 of Figures 1 and 2, respectively) of a medical device according to the invention. For simplicity the housing 7 of the energy supply 5.3 is not depicted. The lithium(-ion) polymer battery of the energy supply 5.3 has a nominal cell voltage of, e.g., 3.7 Volt. The energy supply 5.3 furthermore preferably comprises a fuel gauge unit 10 for estimating and monitoring its remaining running time or life time, respectively. By "fuel" the charge of the energy supply 5.3 or the energy which can be provided by the energy supply 5.3, respectively, is meant. The fuel gauge unit 10 determines the remaining running time via energy or impedance measuring. The fuel gauge unit 10 constitutes so-called "smart" electronics.

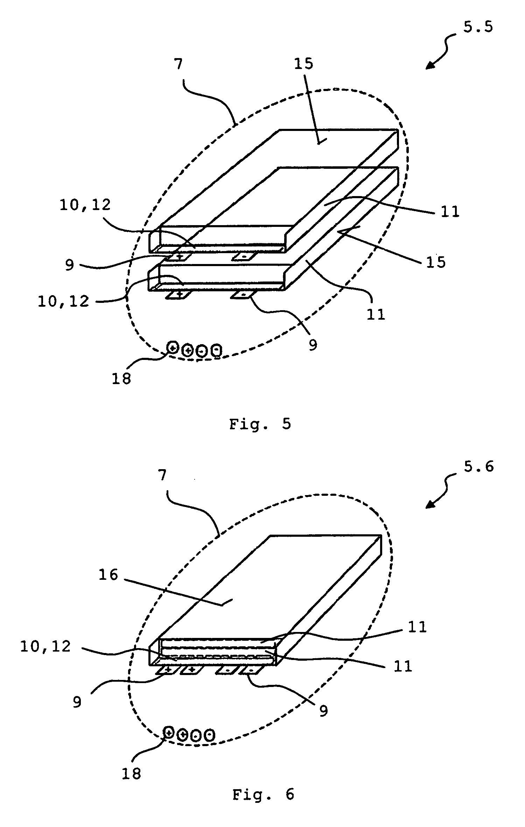

Due to the laminated design of the lithium(-ion) polymer battery which may consist of several battery cells, in particular bi-cells, it is possible to integrate two or more battery stacks, each consisting of battery cells, as independent energy sources in one replaceable unit (namely the energy supply). The lithium(-ion) polymer battery exemplarily comprises two battery stacks 11 with each battery stack 11 representing one energy source (confer Figures 5 to 8). Each energy source/battery stack 11 has a terminal 9 on a positive potential and a terminal 9 on negative potential. With two battery stacks 11 there are therefore four terminals 9. Furthermore, each energy source/battery stack 11 is provided with a protection circuit 12 for protection against overcharge and/or overvoltage and/or undervoltage. There can be provided one fuel gauge unit 10 for the entire energy supply 5.3 or each energy source/battery stack 11 can have its own fuel gauge unit 10 allotted to it. The fuel gauge unit 10 and the protection circuit 12 are preferably enclosed by the housing 7 of the energy supply (confer Figures 5 to 8) and are thus integrated in the energy supply 5.3.

The medical device comprises a reservoir for a fluid. If the medical device is, for example, an insulin pump, it typically comprises as reservoir a cartridge for insulin. Advantageously, the medical device is constructed such that the reservoir is supported and carried by the energy supply. Fig. 4 depicts an energy supply 5.4, which corresponds to the energy supplies 5.1, 5.2 and 5.3 of Figures 1 to 3, that has a cavity 13 for accommodating such a reservoir 14. The cavity 13 is exemplarily formed such that it can accommodate a cylindrically shaped reservoir 14 such as a cartridge of an insulin pump. The reservoir 14 can either be enclosed by the housing of the energy supply 5.4 or it can be held and supported by the housing of the energy supply 5.4, the housing forming the cavity 13.

Of course the energy supply can carry other or further components of the medical device e.g. one or more glucose test strips or a test strip drum. This gives handling advantages as the energy supply and the reservoir and/or other components can be assembled by the user outside the medical device and after assembling be inserted as one unit into the medical device. Referring to Figure 4, before insertion of the energy supply 5.4 into the medical device, the reservoir, in particular a cartridge, a pouch or a bag, can be placed in the cavity 13. If the cavity 13 is formed by the housing of the energy supply 5.4, the housing also serves as support for the reservoir 14 next to its primary function of constituting a housing for the energy supply 5.4.

Other embodiments are possible, where the reservoir (and/or other components of the medical device) carries and supports the energy supply.

By combining the reservoir for fluid and the energy supply with its fuel gauge unit(s) and its protection circuit(s) into one unit, the interfaces for fluid and for electric energy are also combined in this one unit which is advantageous for sealing purposes. The capacity of the reservoir and the running time of the energy supply are time-synchronized such that change or refill of the reservoir is required basically at the same time as change or recharge of the energy supply is required. The remaining running time of the energy supply is thereby estimated by the fuel gauge unit. Hence, the therapeutically necessary handling step of reservoir refill or exchange is combined with the technically required handling step of energy supply exchange or recharge.

Figures 5 to 8 show various embodiments of the energy supply of the medical device. The dashed, elliptical form of the housing 7 has been chosen for simplicity of representation. The terminals 9 of the battery stacks 11 are connected to corresponding contacts 18 of the housing 7.

In Figure 5 an energy supply 5.5 is shown that comprises a rechargeable lithium(-ion) polymer-battery with at least two battery stacks 11 as energy sources as described above. The battery stacks 11 each have their own cover 15, the cover 15 preferentially being made of laminated aluminum foil. The battery stacks 11 are preferably placed on top of each other. The battery stacks 11 are placed in the energy supply housing 7, which is preferably made of plastics. The embodiment of the energy supply 5.5 of Figure 5 is also called fully redundant as the battery stacks 11 and their covers 15 are equally structured. There can be provided one fuel gauge unit 10 for the entire energy supply 5.5 or each energy source/battery stack 11 can have its own fuel gauge unit 10 allotted to it. The fuel gauge unit 10 and the protection circuit 12 are preferably enclosed by the housing 7 of the energy supply and are thus integrated in the energy supply 5.5.

The embodiment shown in Figure 6 differs from the embodiment shown in Figure 5 in that the energy supply 5.6 comprises two battery stacks 11 that are accommodated in/enclosed by one common cover 16, which may be made of laminated aluminum foil. This embodiment is also referred to as quasi redundant.

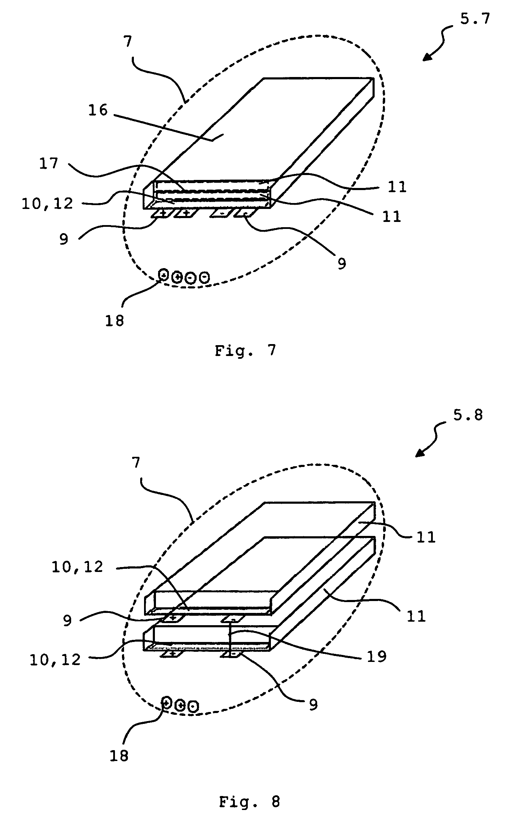

The embodiment of the energy supply 5.7 shown in Figure 7 differs from the embodiment shown in Figure 6 in that the battery stacks 11 are separated by a sealing sheet 17, which may be made of laminated aluminum foil. This embodiment is also referred to as quasi redundant. In Figure 7 the battery stacks 11 are separated by one layer (the sealing sheet 17), while in Figure 5 the battery stacks are separated by two layers (twice the cover 15). Hence, the embodiment according to Figure 7 needs less space than the embodiment according to Figure 5.

Figure 8 shows an energy supply 5.8 which corresponds to the embodiment of Figure 5 but where the terminals 9 on negative potential are connected for all battery stacks/energy sources 11 such that the battery stacks 11 have one common terminal 19 on negative potential. The housing 7 has contacts 18 that correspond to terminals 9 on positive potential and to the common terminal 19 on negative potential and that are connected to them. A connection of the terminals 9 on negative potential for forming one common negative terminal 19 is also possible for the embodiments depicted in Figures 3 to 7.

In the embodiments depicted in Figures 5 to 8 the battery stacks 11 do not necessarily have to be placed on top of each other but can be arranged differently, for example next to each other. The depicted energy supplies 5.5 to 5.8 correspond to the energy supplies 5.1 to 5.4 depicted in Figures 1 to 4. Features of the energy supplies 5.1 to 5.8 may be combined in all possible combinations.

The contacts 18 of the housing 7 that are connected to the terminals 9, 19 of the battery stacks 11 represent the electrical interface of the energy supply 5.1 to 5.8. The output voltage and/or current of these contacts 18 is preferably monitored periodically for fault detection regarding the battery stacks 11, in particular within the multiple error occurrence period. Once a fault has been detected the faulty battery stack 11 is switched off, i.e. disconnected, and its function may be taken over by the other battery stack 11. The multiple error occurrence period is defined as the period of time, in which the probability for the occurrence of multiple errors/faults that are safety critical is sufficiently small for a particular considered requirement class. This period of time starts at the last point in time when the considered device/system has been in a state which supposedly is fault-free according to the considered requirement class. Fault detection in one battery stack 11 and switching entirely to the non-faulty battery stack 11 ensures safe operation at least long enough to trigger and/or produce an alarm in particular for a given time.

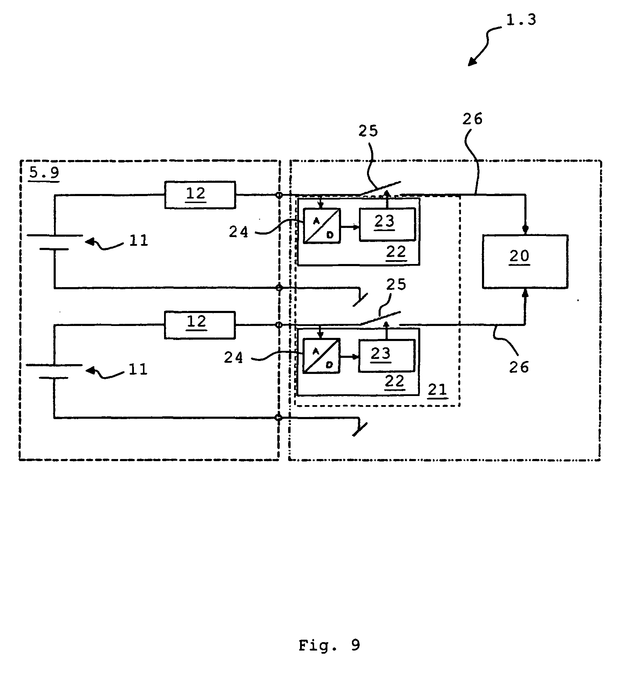

Figure 9 shows a block diagram of a medical device 1.3 according to the invention that corresponds to the medical devices 1.1, 1.2 depicted in Figures 1 and 2. The medical device 1.3 comprises an energy supply 5.9 that corresponds to the energy supplies 5.1 to 5.8 shown in Figures 1 to 8. The energy supply 5.9 comprises a rechargeable lithium(-ion) polymer battery with two battery stacks 11 as described above. Each battery stack 11 is provided with a protection circuit 12. The energy supply 5.9 supplies energy to a load 20, e.g. the pump in case of the medical device 1.3 being an insulin pump. The load 20 can comprise several loads. A control unit 21 is provided with a control subunit 22 for each battery stack 11. The control subunits 22 may be integrated in one unit as control unit 21 or they may be separate units. Each control subunit 22 comprises means for measuring and evaluating (not shown) the output voltage and/or current of the battery stack 11 it is allotted to and means 23 for disconnecting this battery stack 11 from the load 20. Preferably the means for measuring and evaluating comprise an analogue sensor for measuring the output voltage and/or current. The analogue measurement signal is then converted by an analogue-digital converter 24 of the control subunit 22 into a digital signal which can then be evaluated by the means 23 for disconnecting or another evaluation circuit. The means 23 for disconnecting the battery stack 11 preferentially control a switch 25 in the electrical path 26 from the respective battery stack 11 to the load 20. The control unit 21 may also comprise the fuel gauge unit(s) 10 and the protection circuit(s) 12 in particular in case of a fix installed lithium(-ion) polymer battery.

If the means for measuring and evaluating of one of the control subunits 22 detects a fault in the battery stack 11 it is allotted to, then the means 23 for disconnecting controls the switch 25 such that it opens and the path 26 from the faulty battery stack 11 to the load 20 is disconnected. The other non-faulty battery stack 11 is then solely responsible for supplying electrical energy and reliable operation is still ensured as well as alarming. A fault may e.g. be that the respective battery stack 11 provides no output voltage or an output voltage that lies below a pre-defined threshold, the threshold being stored in the control unit 21 or the control subunits 22, or a failure of a battery connection, respectively.

The energy distribution among the battery stacks 11 may, for example, be 50/50, i.e. each battery stack 11 provides 50 percent of electrical energy required by the load 20. If the load 20 comprises several subloads, these subloads may be allotted accordingly to the battery stacks 11. Of course, the energy distribution among the battery stacks 11 may be differently and asymmetrically, e.g. 95/5, where one battery stack 11 provides 95 percent of the electrical energy and the other battery stack only provides 5 percent, thereby basically serving as emergency energy supply for triggering an alarm in case of a fault. Preferentially the electronics (not shown) of the load 20 is designed such that it can alternatively access each or both of the battery stacks 11 while determining their respective output voltages.

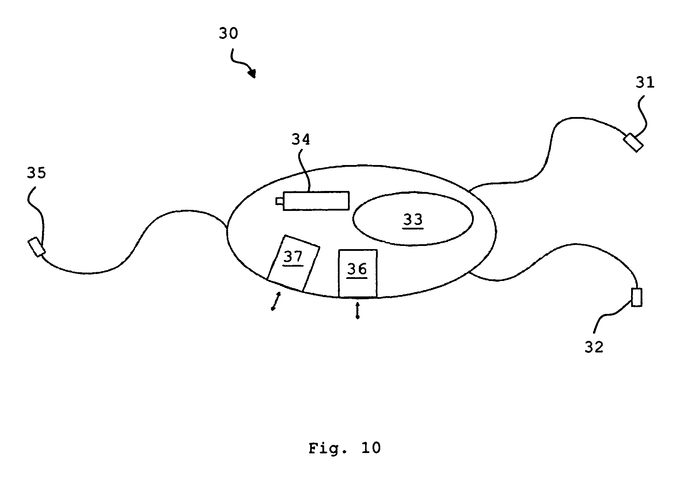

Figure 10 shows an exemplary charging station 30 for charging an energy supply 5.1 to 5.9 of a medical device 1.1, 1.2, 1.3, the charging station 30 and a medical device 1.1, 1.2, 1.3 as described above, in combination, making a medical system. The charging station 30 comprises as energy supplying means an USB-plug 31, a line connector/network connector 32 in form of a 110-230 VAC/DC plug (110 to 230 Volts of alternating current to direct current plug), a solar cell panel 32, a primary battery 34 (e.g. an AA battery, an AAA battery and/or a 9 Volt block battery) and a plug 35 for a car cigarette lighter outlet (i.e. an 12 Volts of direct current (VDC) car plug). A charge slot 36 for the energy supply 5.1 to 5.9 is provided which is connected or connectable to the various energy supplying means 31 to 35 via a charging circuit (not shown).

By providing several different types of energy supplying means, the user is not restricted to one type of energy (e.g. a primary battery) but can switch to a different type of energy (e.g. solar energy if the user is outside or energy provided by a car battery if he is in a car) which is available at the particular moment. This enhances the independency of the user from purchasable primary batteries and guaranties substantially permanent and uninterruptible energy availability also in non-everyday situations like on vacation or on travel.

The charging station 30 preferentially comprises also a storage slot 37 for storing an energy supply 5.1 to 5.9, that shall not be used for some time, such that it remains in optimal charge condition, in particular basically fully charged. The storage slot 37 is connected or connectable to the various energy supplying means 31 to 35. By storing an energy supply 5.1 to 5.9 in the storage slot 37 its nominal cell voltage is preferably kept between 3.0 and 3.7 Volt through charge preservation, thereby basically guaranteeing optimal storage condition. The electrical energy required for maintaining this optimal storage voltage is provided by the various energy supplying means 31 to 35 of the charging station. A control circuit (not shown) is allotted to the storage slot 37 for controlling the charge of the stored energy supply 5.1 to 5.9 such that its nominal cell voltage preferably does not fall below 3.0 Volt and is within the range of the optimal storage voltage of 3.7 Volt. Another alternative to ensure a proper charge condition may be realized by the charging circuit itself. For this alternative the charging circuit is preferably constructed such that it is able to supervise the voltage of the energy supply 5.1 to 5.9 after having it charged in order to ensure proper/optimal storage condition.

The charging station 30 preferentially has a display (not shown) for displaying the actual charge of an energy supply inserted into the charge slot 36 and/or the storage slot 37. More than one charge slot 36 and/or storage slot 37 may be provided. Preferably there is one display for each slot 36, 37. The respective displays are in particular arranged directly above the corresponding slots 36, 37 and, hence, the corresponding energy supplies 5.1 to 5.9 inserted into the slots 36, 37, so that it is easy for the user to pick the optimally charged energy supply 5.1 to 5.9 for usage.

## Claims

1. Medical device with an energy supply (5.1; 5.2; 5.3; 5.4; 5.5; 5.6; 5.7; 5.8; 5.9), the energy supply (5.1; 5.2; 5.3; 5.4; 5.5; 5.6; 5.7; 5.8; 5.9) comprising at least two energy sources (11), of which at least one energy source (11) constitutes a backup energy source, and with a control unit (21) for monitoring and controlling the energy supply (5.1; 5.2; 5.3; 5.4; 5.5; 5.6; 5.7; 5.8; 5.9), the control unit (21) being designed such that it can detect a fault in the energy sources (11) and disconnect the faulty energy source (11), and the energy supply (5.1; 5.2; 5.3; 5.4; 5.5; 5.6; 5.7; 5.8; 5.9) is rechargeable and comprises a lithium(-ion) polymer battery with at least two battery stacks (11), each battery stack constituting one energy source, having a terminal (9) on positive potential and a terminal (9) on negative potential and comprising one or more battery cells, wherein the energy supply (5.1; 5.2; 5.3; 5.4; 5.5; 5.6; 5.7; 5.8; 5.9) comprises a fuel gauge unit (10) for estimating its remaining running time,
**characterized in that** a reservoir (14) for a fluid is provided and the capacity of the reservoir (14) and the running time of the energy supply (5.1; 5.2; 5.3; 5.4; 5.5; 5.6; 5.7; 5.8; 5.9) are synchronized such that change or refill of the reservoir (14) is required at the same time as change or recharge of the energy supply (5.1; 5.2; 5.3; 5.4; 5.5; 5.6; 5.7; 5.8; 5.9).

2. Medical device according to claim 1, wherein each battery stack (11) has its own cover (15).

3. Medical device according to claim 1, wherein the battery stacks (11) are accommodated in one cover (16).

4. Medical device according to claim 3, wherein the battery stacks (11) are separated by a sealing sheet (17).

5. Medical device according to either of the preceding claims, wherein the terminals (9) on negative potentials are connected for all energy sources (11), such that the energy sources (11) have one common terminal (19) on negative potential.

6. Medical device according to one of the preceding claims, wherein the battery stacks (11) are positioned side-by-side or on top of each other.

7. Medical device according to one of the preceding claims, wherein the energy sources (11) are accommodated in a common housing (7).

8. Medical device according to one of the preceding claims, wherein the energy supply (5.1; 5.2; 5.3; 5.4; 5.5; 5.6; 5.7; 5.8; 5.9) is replaceable or fixed in the medical device (1.1; 1.2; 1.3).

9. Medical device according to one of the preceding claims, wherein the control unit (21) comprises means for measuring and evaluating the output voltage and/or current of each energy source (11) and means (23) for disconnecting each energy source (11).

10. Medical device according to claim 9, wherein the control unit (21) comprises a control subunit (22) for each energy source (11), each control subunit (22) having means for measuring and evaluating the output voltage and/or current of its associated energy source (11) and means (23) for disconnecting the associated energy source (11).

11. Medical device according to one of the preceding claims, wherein each energy source (11) is provided with a protection circuit (12) for protection from overcharge and/or overvoltage and/or undervoltage.

12. Medical device according to one of the preceding claims, wherein the reservoir (14) is carried by the energy supply (5.1; 5.2; 5.3; 5.4; 5.5; 5.6; 5.7; 5.8; 5.9).

13. Medical device according to claim 12, wherein the energy supply (5.1; 5.2; 5.3; 5.4; 5.5; 5.6; 5.7; 5.8; 5.9) has a cavity (13) for accommodating the reservoir (14), the cavity (13) preferably being formed to accommodate a cylindrically shaped reservoir (14) or a bag.

14. Medical device according to claim 12 or 13, wherein the reservoir (14) is enclosed by a housing (7) of the energy supply (5.1; 5.2; 5.3; 5.4; 5.5; 5.6; 5.7; 5.8; 5.9) or the reservoir (14) is carried by a housing (7) of the energy supply (5.1; 5.2; 5.3; 5.4; 5.5; 5.6; 5.7; 5.8; 5.9).

15. Medical device according to one of the preceding claims, wherein at least a part of a housing (3.2) of the medical device (1.1; 1.2; 1.3) is formed by a housing (7) of the energy supply (5.1; 5.2; 5.3; 5.4; 5.5; 5.6; 5.7; 5.8; 5.9).

16. Medical device according to one of the preceding claims, wherein contact surfaces (8) of the energy supply (5.1; 5.2; 5.3; 5.4; 5.5; 5.6; 5.7; 5.8; 5.9) are provided with gaskets.

17. Medical system comprising a medical device according to one of the preceding claims and further comprising a charging station (30) for an energy supply (5.1; 5.2; 5.3; 5.4; 5.5; 5.6; 5.7; 5.8; 5.9) of the medical device (1.1; 1.2; 1.3), wherein as energy supplying means an USB-plug (31), a line connector (32), a solar cell panel (33), a primary battery (34), a plug (35) for a car cigarette lighter outlet, a connector for a mobile phone accumulator and/or a dynamo with a crank handle are provided.

18. Medical system according to Claim 17, wherein a storage slot (37) is provided by the charging station (30) for storing the energy supply (5.1; 5.2; 5.3; 5.4; 5.5; 5.6; 5.7; 5.8; 5.9) such that it remains basically fully charged.

## Patentansprüche

1. Medizinprodukt mit einer Energieversorgung (5.1; 5.2; 5.3; 5.4; 5.5; 5.6; 5.7; 5.8; 5.9), wobei die Energieversorgung (5.1; 5.2; 5.3; 5.4; 5.5; 5.6; 5.7; 5.8; 5.9) mindestens zwei Energiequellen (11) umfasst, von denen mindestens eine Energiequelle (11) eine Reserveenergiequelle darstellt, und mit einer Steuereinheit (21) zum Überwachen und Steuern der Energieversorgung (5.1; 5.2; 5.3; 5.4; 5.5; 5.6; 5.7; 5.8; 5.9), wobei die Steuereinheit (21) derart ausgestaltet ist, dass sie einen Fehler bei den Energiequellen (11) erfassen und die fehlerhafte Energiequelle (11) abschalten kann, und wobei die Energieversorgung (5.1; 5.2; 5.3; 5.4; 5.5; 5.6; 5.7; 5.8; 5.9) wiederaufladbar ist und eine Lithium(-ionen)-Polymerbatterie mit mindestens zwei Batteriestapeln (11) umfasst, wobei jeder Batteriestapel eine Energiequelle darstellt, die einen Anschluss (9) am positiven Potential und einen Anschluss (9) am negativen Potential aufweist und eine oder mehrere Batteriezellen umfasst, wobei die Energieversorgung (5.1; 5.2; 5.3; 5.4; 5.5; 5.6; 5.7; 5.8; 5.9) eine Ladungsanzeigeeinheit (10) zum Abschätzen ihrer verbleibenden Laufzeit umfasst,
**dadurch gekennzeichnet, dass** ein Reservoir (14) für eine Flüssigkeit bereitgestellt ist und die Kapazität des Reservoirs (14) und die Laufzeit der Energieversorgung (5.1; 5.2; 5.3; 5.4; 5.5; 5.6; 5.7; 5.8; 5.9) derart synchronisiert sind, dass ein Austausch oder Auffüllen des Reservoirs (14) zu der gleichen Zeit wie ein Austausch oder Aufladen der Energieversorgung (5.1; 5.2; 5.3; 5.4; 5.5; 5.6; 5.7; 5.8; 5.9) erforderlich ist.

2. Medizinprodukt nach Anspruch 1, wobei jeder Batteriestapel (11) seine eigene Abdeckung (15) aufweist.

3. Medizinprodukt nach Anspruch 1, wobei die Batteriestapel (11) in einer Abdeckung (16) aufgenommen sind.

4. Medizinprodukt nach Anspruch 3, wobei die Batteriestapel (11) durch eine Dichtungsfolie (17) getrennt sind.

5. Medizinprodukt nach einem der vorstehenden Ansprüche, wobei die Anschlüsse (9) an negativen Potentialen für alle Energiequellen (11) derart verbunden sind, dass die Energiequellen (11) einen gemeinsamen Anschluss (19) am negativen Potential aufweisen.

6. Medizinprodukt nach einem der vorstehenden Ansprüche, wobei die Batteriestapel (11) Seite an Seite oder übereinander positioniert sind.

7. Medizinprodukt nach einem der vorstehenden Ansprüche, wobei die Energiequellen (11) in einem gemeinsamen Gehäuse (7) aufgenommen sind.

8. Medizinprodukt nach einem der vorstehenden Ansprüche, wobei die Energieversorgung (5.1; 5.2; 5.3; 5.4; 5.5; 5.6; 5.7; 5.8; 5.9) austauschbar oder im Medizinprodukt (1.1; 1.2; 1.3) fixiert ist.

9. Medizinprodukt nach einem der vorstehenden Ansprüche, wobei die Steuereinheit (21) Mittel zum Messen und Auswerten der Ausgangsspannung und/oder des -stroms jeder Energiequelle (11) und Mittel (23) zum Abschalten jeder Energiequelle (11) umfasst.

10. Medizinprodukt nach Anspruch 9, wobei die Steuereinheit (21) eine Steueruntereinheit (22) für jede Energiequelle (11) umfasst, wobei jede Steueruntereinheit (22) Mittel zum Messen und Auswerten der Ausgangsspannung und/oder des -stroms ihrer zugehörigen Energiequelle (11) und Mittel (23) zum Abschalten der zugehörigen Energiequelle (11) aufweist.

11. Medizinprodukt nach einem der vorstehenden Ansprüche, wobei jede Energiequelle (11) mit einer Schutzschaltung (12) zum Schutz vor Überladung und/oder Überspannung und/oder Unterspannung bereitgestellt ist.

12. Medizinprodukt nach einem der vorstehenden Ansprüche, wobei das Reservoir (14) von der Energieversorgung (5.1; 5.2; 5.3; 5.4; 5.5; 5.6; 5.7; 5.8; 5.9) getragen wird.

13. Medizinprodukt nach Anspruch 12, wobei die Energieversorgung (5.1; 5.2; 5.3; 5.4; 5.5; 5.6; 5.7; 5.8; 5.9) einen Hohlraum (13) zum Aufnehmen des Reservoirs (14) aufweist, wobei der Hohlraum (13) vorzugsweise ausgebildet ist, um ein zylindrisch gestaltetes Reservoir (14) oder einen Beutel aufzunehmen.

14. Medizinprodukt nach Anspruch 12 oder 13, wobei das Reservoir (14) von einem Gehäuse (7) der Energieversorgung (5.1; 5.2; 5.3; 5.4; 5.5; 5.6; 5.7; 5.8; 5.9) umschlossen ist oder das Reservoir (14) von einem Gehäuse (7) der Energieversorgung (5.1; 5.2; 5.3; 5.4; 5.5; 5.6; 5.7; 5.8; 5.9) getragen wird.

15. Medizinprodukt nach einem der vorstehenden Ansprüche, wobei mindestens ein Teil eines Gehäuses (3.2) des Medizinprodukts (1.1; 1.2; 1.3) von einem Gehäuse (7) der Energieversorgung (5.1; 5.2; 5.3; 5.4; 5.5; 5.6; 5.7; 5.8; 5.9) gebildet wird.

16. Medizinprodukt nach einem der vorstehenden Ansprüche, wobei Kontaktflächen (8) der Energieversorgung (5.1; 5.2; 5.3; 5.4; 5.5; 5.6; 5.7; 5.8; 5.9) mit Dichtungen bereitgestellt sind.

17. Medizinsystem, umfassend ein Medizinprodukt nach einem der vorstehenden Ansprüche und ferner umfassend eine Ladestation (30) für eine Energieversorgung (5.1; 5.2; 5.3; 5.4; 5.5; 5.6; 5.7; 5.8; 5.9) des Medizinprodukts (1.1; 1.2; 1.3), wobei als Energieversorgungsmittel ein USB-Stecker (31), ein Leitungsverbinder (32), ein Solarzellenpanel (33), eine Primärbatterie (34), ein Stecker (35) für einen Ausgang eines Auto-Zigarettenanzünders, ein Verbinder für einen Handy-Akkumulator und/oder ein Dynamo mit einer Handkurbel bereitgestellt sind.

18. Medizinsystem nach Anspruch 17, wobei ein Aufbewahrungsschlitz (37) von der Ladestation (30) zum Aufbewahren der Energieversorgung (5.1; 5.2; 5.3; 5.4; 5.5; 5.6; 5.7; 5.8; 5.9) bereitgestellt ist, so dass sie im Wesentlichen vollständig aufgeladen bleibt.

## Revendications

1. Dispositif médical ayant une alimentation en énergie (5.1 ; 5.2 ; 5.3 ; 5.4 ; 5.5 ; 5.6 ; 5.7 ; 5.8 ; 5.9), l'alimentation en énergie (5.1 ; 5.2 ; 5.3 ; 5.4 ; 5.5 ; 5.6 ; 5.7 ; 5.8 ; 5.9) comprenant au moins deux sources d'énergie (11), dont au moins une source d'énergie (11) constitue une source d'énergie de secours, et ayant une unité de commande (21) pour surveiller et commander l'alimentation en énergie (5.1 ; 5.2 ; 5.3 ; 5.4 ; 5.5 ; 5.6 ; 5.7 ; 5.8 ; 5.9), l'unité de commande (21) étant agencée de telle sorte qu'elle peut détecter une défaillance dans les sources d'énergie (11) et déconnecter la source d'énergie (11) défaillante, et l'alimentation en énergie (5.1 ; 5.2 ; 5.3 ; 5.4 ; 5.5 ; 5.6 ; 5.7 ; 5.8 ; 5.9) est rechargeable et comporte une batterie lithium(-ion) polymère ayant au moins deux empilements de batterie (11), chaque empilement de batterie constituant une source d'énergie, ayant une borne (9) sur un potentiel positif et une borne (9) sur un potentiel négatif et comprenant une ou plusieurs cellules de batterie, l'alimentation en énergie (5.1 ; 5.2 ; 5.3 ; 5.4 ; 5.5 ; 5.6 ; 5.7 ; 5.8 ; 5.9) comprenant une unité de jauge de combustible (10) pour estimer son temps de fonctionnement restant,
**caractérisé par le fait qu'**un réservoir (14) pour un fluide est prévu et la capacité du réservoir (14) et le temps de fonctionnement de l'alimentation en énergie (5.1 ; 5.2 ; 5.3 ; 5.4 ; 5.5 ; 5.6 ; 5.7 ; 5.8 ; 5.9) sont synchronisés de telle sorte qu'un changement ou une recharge du réservoir (14) est requis au même moment qu'un changement ou une recharge de l'alimentation en énergie (5.1 ; 5.2 ; 5.3 ; 5.4 ; 5.5 ; 5.6 ; 5.7 ; 5.8 ; 5.9).

2. Dispositif médical selon la revendication 1, dans lequel chaque empilement de batterie (11) a son propre couvercle (15).

3. Dispositif médical selon la revendication 1, dans lequel les empilements de batterie (11) sont reçus dans un couvercle (16).

4. Dispositif médical selon la revendication 3, dans lequel les empilements de batterie (11) sont séparés par une feuille de scellement étanche (17).

5. Dispositif médical selon l'une des revendications précédentes, dans lequel les bornes (9) sur des potentiels négatifs sont connectées pour toutes les sources d'énergie (11), de telle sorte que les sources d'énergie (11) ont une borne commune (19) sur un potentiel négatif.

6. Dispositif médical selon l'une des revendications précédentes, dans lequel les empilements de batterie (11) sont positionnés côte à côte ou les uns sur les autres.

7. Dispositif médical selon l'une des revendications précédentes, dans lequel les sources d'énergie (11) sont reçues dans un boîtier commun (7).

8. Dispositif médical selon l'une des revendications précédentes, dans lequel l'alimentation en énergie (5.1 ; 5.2 ; 5.3 ; 5.4 ; 5.5 ; 5.6 ; 5.7 ; 5.8 ; 5.9) est remplaçable ou fixe dans le dispositif médical (1.1 ; 1.2 ; 1.3).

9. Dispositif médical selon l'une des revendications précédentes, dans lequel l'unité de commande (21) comprend des moyens pour mesurer et évaluer la tension de sortie et/ou le courant de sortie de chaque source d'énergie (11) et des moyens (23) pour déconnecter chaque source d'énergie (11).

10. Dispositif médical selon la revendication 9, dans lequel l'unité de commande (21) comprend une sous-unité de commande (22) pour chaque source d'énergie (11), chaque sous-unité de commande (22) ayant des moyens pour mesurer et évaluer la tension de sortie et/ou le courant de sortie de sa source d'énergie (11) associée et des moyens (23) pour déconnecter la source d'énergie (11) associée.

11. Dispositif médical selon l'une des revendications précédentes, dans lequel chaque source d'énergie (11) comporte un circuit de protection (12) pour une protection vis-à-vis d'une surcharge et/ou d'une surtension et/ou d'une sous-tension.

12. Dispositif médical selon l'une des revendications précédentes, dans lequel le réservoir (14) est porté par l'alimentation en énergie (5.1 ; 5.2 ; 5.3 ; 5.4 ; 5.5 ; 5.6 ; 5.7 ; 5.8 ; 5.9).

13. Dispositif médical selon la revendication 12, dans lequel l'alimentation en énergie (5.1 ; 5.2 ; 5.3 ; 5.4 ; 5.5 ; 5.6 ; 5.7 ; 5.8 ; 5.9) a une cavité (13) pour recevoir le réservoir (14), la cavité (13) étant, de préférence, formée pour recevoir un réservoir (14) de forme cylindrique ou un sac.

14. Dispositif médical selon l'une des revendications 12 ou 13, dans lequel le réservoir (14) est enfermé par un boîtier (7) de l'alimentation en énergie (5.1 ; 5.2 ; 5.3 ; 5.4 ; 5.5 ; 5.6 ; 5.7 ; 5.8 ; 5.9) ou le réservoir (14) est porté par un boîtier (7) de l'alimentation en énergie (5.1 ; 5.2 ; 5.3 ; 5.4 ; 5.5 ; 5.6 ; 5.7 ; 5.8 ; 5.9).

15. Dispositif médical selon l'une des revendications précédentes, dans lequel au moins une partie d'un boîtier (3.2) du dispositif médical (1.1 ; 1.2 ; 1.3) est formée par un boîtier (7) de l'alimentation en énergie (5.1 ; 5.2 ; 5.3 ; 5.4 ; 5.5 ; 5.6 ; 5.7 ; 5.8 ; 5.9).

16. Dispositif médical selon l'une des revendications précédentes, dans lequel des surfaces de contact (8) de l'alimentation en énergie (5.1 ; 5.2 ; 5.3 ; 5.4 ; 5.5 ; 5.6 ; 5.7 ; 5.8 ; 5.9) sont munies de joints d'étanchéité.

17. Système médical comprenant un dispositif médical selon l'une des revendications précédentes et comprenant en outre une station de charge (30) pour une alimentation en énergie (5.1 ; 5.2 ; 5.3 ; 5.4 ; 5.5 ; 5.6 ; 5.7 ; 5.8 ; 5.9) du dispositif médical (1.1 ; 1.2 ; 1.3), dans lequel sont prévus, en tant que moyens d'alimentation en énergie, une fiche USB (31), un connecteur réseau (32), un panneau de cellules solaires (33), une batterie primaire (34), une fiche (35) pour une sortie d'allume-cigare de voiture, un connecteur pour un accumulateur de téléphone mobile et/ou une génératrice à courant continu avec une manivelle.

18. Système médical selon la revendication 17, dans lequel une fente de stockage (37) est fournie par la station de charge (30) pour stocker l'alimentation en énergie (5.1 ; 5.2 ; 5.3 ; 5.4 ; 5.5 ; 5.6 ; 5.7 ; 5.8 ; 5.9), de telle sorte qu'elle reste essentiellement chargée complètement.
